# EUROPEAN PATENT APPLICATION

(11) **EP 4 585 178 A1**
(43) Date of publication of application: **16.07.2025**
(21) Application number: 23863235.0
(22) Date of filing: 07.09.2023
(51) Int. Cl.: A61B 34/00, G06N 20/10

(54) **SURGERY ASSISTANCE PROGRAM, SURGERY ASSISTANCE DEVICE, AND SURGERY ASSISTANCE METHOD**

(30) Priority: 09.09.2022 JP 2022143971
(71) Applicant: Keio University, Tokyo, 108-8345 (JP)
(72) Inventor: TAKEUCHI, Masashi, Tokyo 160-8582 (JP)
(74) Representative: Epping - Hermann - Fischer
(86) International application number: PCT/JP2023/032610
(87) International publication number: WO 2024/053697

(57) **Abstract**

Notification of a state of resection of a resection target is accurately provided during surgery.

A program according to an embodiment of the present invention causes a computer to: acquire a surgery image in which a surgical site is imaged during surgery for resecting a resection target; detect a state of resection of the resection target from the acquired surgery image by using a machine learning model generated by training with training data in which a surgery image is an input and information related to completion of resection is an output; and notify a state of resection of the resection target, wherein the information related to completion of resection includes region information of an organ that appears in the surgery image when the resection target is resected.

## Description

### TECHNICAL FIELD

The present invention relates to a surgery assistance program, a surgery assistance apparatus, and a surgery assistance method.

### BACKGROUND ART

Conventionally, surgical operations, particularly operations for malignant tumors, require sufficient resection (also called "dissection") of not only a tumor itself but also lymph nodes around the tumor. Insufficient resection causes postoperative recurrence and consequently worsens the patient's long-term prognosis. Excessive resection, on the other hand, may cause increased postoperative complications.

As a technique for assisting appropriate resection of a resection target, for example, Patent Document 1 discloses a near-infrared fluorescent tracer and a fluorescence imaging method. Specifically, Patent Document 1 describes that in vitro real-time measurement of the position and size of a tumor in a living body can be realized by chemically binding an anti-tumor antibody to an indocyanine green-high density lipoprotein (ICG-HDL) complex. However, with the method of Patent Document 1, the operator can only distinguish between tumor tissue and normal tissue, and the determination of completion of resection is left to the operator.

As a method of notifying completion of a procedure using machine learning, for example, Non-Patent Document 1 discloses a method of training a machine learning model with surgery images and information regarding completion of the procedure and outputting a state of completion of the procedure in response to an input of a surgery image at the time of inference.

### RELATED ART DOCUMENTS

### PATENT DOCUMENT

Patent Document 1: Japanese Unexamined Patent Publication No. H9(1997)-309845

Non-Patent Document 1: Pietro Mascagni, et al. "Artificial Intelligence for Surgical Safety: Automatic Assessment of the Critical View of Safety in Laparoscopic Cholecystectomy Using Deep Learning." Annals of Surgery, vol. 275, no. 5, May 2022, pp. 955-961.

### SUMMARY OF THE INVENTION

### PROBLEMS TO BE SOLVED BY THE INVENTION

In Non-Patent Document 1, however, inference is made through learning of surgery images and information regarding completion of a procedure, and it has been desired to be able to more accurately provide notification about the state of resection of a resection target during a surgical operation.

Therefore, an object of the present invention is to accurately provide notification about a state of resection of a resection target during surgery.

### MEANS FOR SOLVING THE PROBLEMS

A program according to an embodiment of the present invention causes a computer to acquire a surgery image in which a surgical site is imaged during surgery for resecting a resection target; detect a state of resection of the resection target from the acquired surgery image by using a machine learning model generated by training with training data in which a surgery image is an input and information related to completion of resection is an output; and notify a state of resection of the resection target, wherein the information related to completion of resection includes region information of an organ that appears in the surgery image when the resection target is resected.

According to the present invention, it is possible to accurately provide notification about the state of resection of a resection target during surgery.

### BRIEF DESCRIPTION OF THE DRAWINGS

[FIG. 1] FIG. 1 is a diagram showing an overall configuration of an embodiment of the present invention.
[FIG. 2] FIG. 2 is a functional block diagram of a surgery assistance apparatus according to an embodiment of the present invention.
[FIG. 3] FIG. 3 is a diagram for explaining region information and labels of an organ according to an embodiment of the present invention.
[FIG. 4] FIG. 4 is an example of organs that appear in a surgery image when a resection target is resected according to an embodiment of the present invention.
[FIG. 5] FIG. 5 is an example of organs that appear in a surgery image when a resection target is resected according to an embodiment of the present invention.
[FIG. 6] FIG. 6 is an example of organs that appear in a surgery image when a resection target is resected according to an embodiment of the present invention.
[FIG. 7] FIG. 7 is an example of organs that appear in a surgery image when a resection target is resected according to an embodiment of the present invention.
[FIG. 8] FIG. 8 is a diagram for explaining machine learning according to an embodiment of the present invention.
[FIG. 9] FIG. 9 is a diagram for explaining machine learning according to an embodiment of the present invention.
[FIG. 10] FIG. 10 is a flowchart of a machine learning process according to an embodiment of the present invention.
[FIG. 11] FIG. 11 is a flowchart of a resection state notification process according to an embodiment of the present invention.
[FIG. 12] FIG. 12 is a flowchart of an uncompleted resection notification process according to an embodiment of the present invention.
[FIG. 13] FIG. 13 is a flowchart of an evaluation process according to an embodiment of the present invention.
[FIG. 14] FIG. 14 is an example of information on which evaluation is based, and evaluation results, according to an embodiment of the present invention.
[FIG. 15] FIG. 15 is a hardware configuration diagram of a surgery assistance apparatus according to an embodiment of the present invention.

### DESCRIPTION OF EMBODIMENTS

Hereinafter, embodiments of the invention will be described with reference to the drawings.

### <Explanation of Terms>

- In the present specification, "surgery" may be any surgery, such as surgery performed only by a human, surgery performed by a human operating a medical device such as an endoscope (for example, laparoscopic surgery, thoracoscopic surgery, or the like), surgery performed by a human operating a robot, or surgery performed only by a robot. For example, surgery is a surgical operation that removes cancerous tissue, including primary lesions and lymph nodes, from tissue. For example, the surgery is esophagectomy, gastrectomy, colectomy, prostatectomy, pancreatectomy, but may also be endoscopic submucosal dissection for early cancer.
- In the present specification, "resection target" is an object (for example, a lesion) to be removed in surgery. For example, a resection target is a malignant tumor and a lymph node around the tumor, but may be a benign tumor.
- In the present specification, "organ" is any organ of the body.

### <System Configuration>

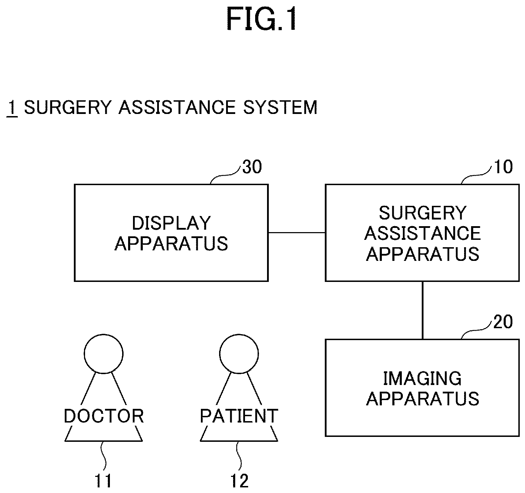

FIG. 1 is a diagram showing an overall configuration of an embodiment of the present invention. The surgery assistance system 1 includes a surgery assistance apparatus 10, an imaging apparatus 20, and a display apparatus 30. A case of surgery in which a doctor 11 removes a resection target of a patient 12 will be described. Each of these will be described below.

### <<Surgery Assistance Apparatus>>

The surgery assistance apparatus 10 is an apparatus that detects a state of resection of a resection target on the basis of an image captured by the imaging apparatus 20 and notifies the doctor 11 of the state of resection of the resection target (for example, causes the display apparatus 30 to display the state) during surgery in which the doctor 11 removes the resection target of the patient 12. The surgery assistance apparatus 10 includes one or a plurality of computers. The surgery assistance apparatus 10 can acquire an image captured by the imaging apparatus 20 from the imaging apparatus 20. The surgery assistance apparatus 10 can cause the display apparatus 30 to display information, such as the state of resection of the resection target.

### <<Imaging Apparatus>>

The imaging apparatus 20 is an apparatus that images a body part of the patient 12 on which surgery is being performed (hereinafter, also referred to as a "surgical site"). Hereinafter, the image of the surgical site captured by the imaging apparatus 20 is also referred to as a "surgery image".

### <<Display Apparatus>>

The display apparatus 30 is a monitor, a display, or the like that displays information acquired from the surgery assistance apparatus 10. The doctor 11 can know the state of resection of the resection target (for example, a completion of resection) by viewing the information displayed on the display apparatus 30.

At least two of the surgery assistance apparatus 10, the imaging apparatus 20, and the display apparatus 30 may be implemented by one device.

### <Functional Blocks>

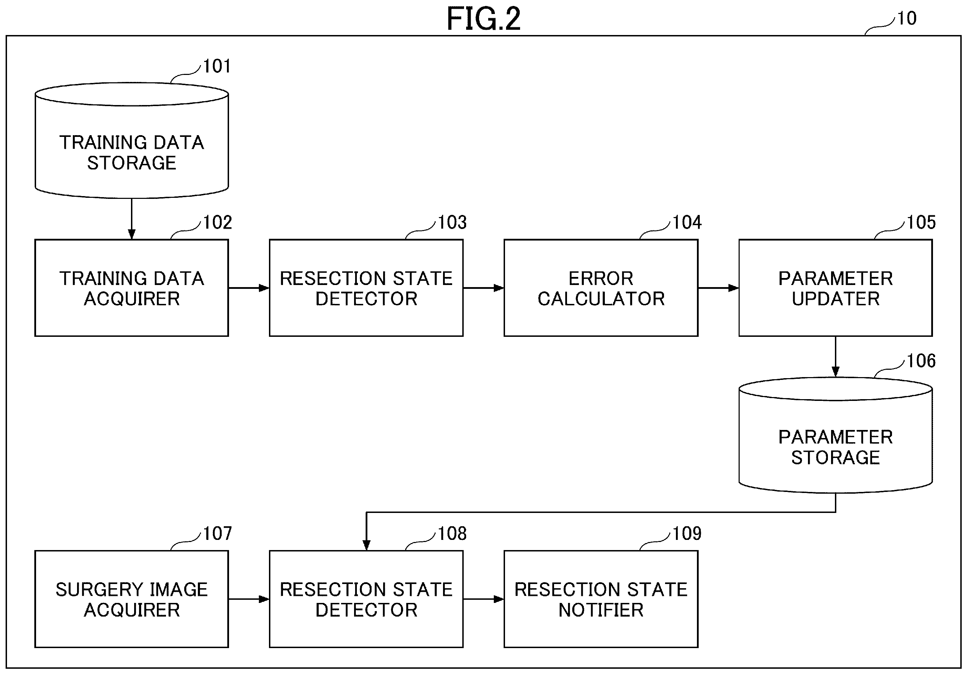

FIG. 2 is a functional block diagram of the surgery assistance apparatus 10 according to an embodiment of the present invention. The surgery assistance apparatus 10 includes a training data storage 101, a training data acquirer 102, a resection state detector 103, an error calculator 104, a parameter updater 105, a parameter storage 106, a surgery image acquirer 107, a resection state detector 108, and a resection state notifier 109. The surgery assistance apparatus 10 executes programs to function as the training data acquirer 102, the resection state detector 103, the error calculator 104, the parameter updater 105, the surgery image acquirer 107, the resection state detector 108, and the resection state notifier 109. Each of these will be described below.

The training data storage 101 stores training data. The training data is a surgery image in which a surgical site is imaged and information related to completion of resection for the surgery image.

The training data acquirer 102 acquires training data stored in the training data storage 101.

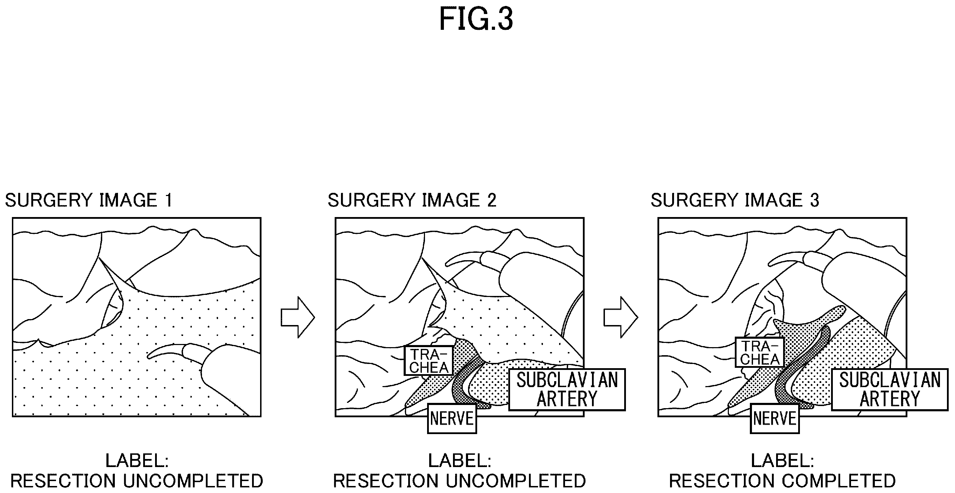

Hereinafter, the "information related to completion of resection" will be explained. The "information related to completion of resection" includes "region information of an organ that appears in the surgery image when the resection target is resected (the region information is at least one of segmentation information indicating the region of the organ in the image (information indicating the segmented organ) or frame information indicating the region of the organ in the image (information such as a rectangle surrounding the organ))" and "a label indicating whether the surgery image is an image in which resection of the resection target is completed or an image in which resection of the resection target is not completed". The region information and the label of the organ will be described in detail below with reference to FIG. 3.

FIG. 3 is a diagram for explaining the region information and the label of an organ according to an embodiment of the present invention. For example, assume that "trachea", "subclavian artery", and "nerve" appear in the surgery image when the resection target is resected. The surgery images 1 to 3 are examples of training data (correct answer data). As the training data, the region information of the resection target (at least one of segmentation information indicating a region of the resection target in the image (information indicating the segmented resection target) and frame information indicating a region of the resection target in the image (information of a rectangle or the like surrounding the resection target)) may also be used.

The surgery image 1 is an image of a surgical site before the resection target is resected. In the surgery image 1, the organs ("trachea", "subclavian artery", and "nerve" in the example of FIG. 3) do not appear yet. The label of the surgery image 1 indicates that the surgery image is an image in which the resection of the resection target is not completed.

The surgery image 2 is an image of a surgical site during the resection of the resection target. In the surgery image 2, region information of the organs that appear during the resection is added. The label of the surgery image 2 indicates that the surgery image is an image in which the resection of the resection target is not completed.

The surgery image 3 is an image of a surgical site after the resection target is resected. In the surgery image 3, region information of the organs that appear after the resection is completed is added. The label of the surgery image 3 indicates that the surgery image is an image in which the resection of the resection target is completed. A point of time when an organ that is to appear in the surgery image at the time of completion of resection is recognized is defined as when the resection is completed.

The region information of an organ may be generated by a human designating the region of the organ in the surgery image, or through inference using a machine learning model trained with surgery images in which regions of organs are designated by a human.

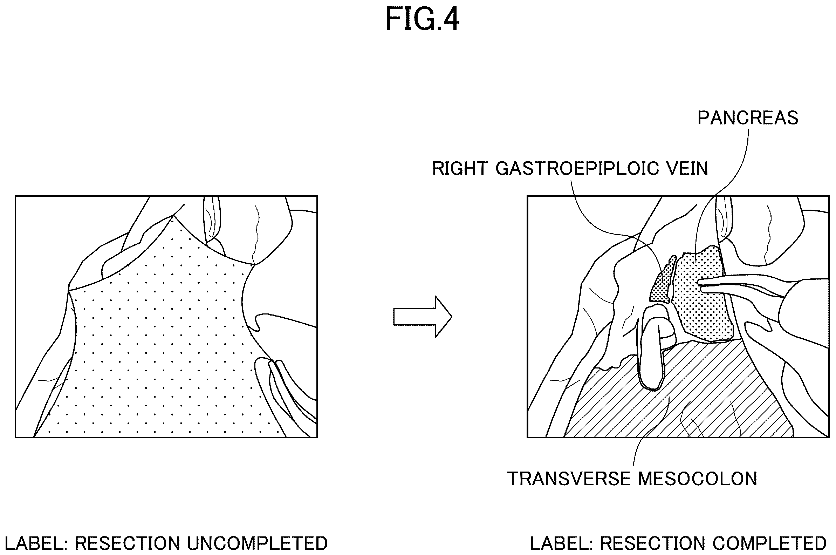

FIG. 4 is an example of the organs that appear in the surgery image when the resection target is resected according to an embodiment of the present invention. In a gastrectomy, when the resection target (tumor and lymph node) is resected, the transverse mesocolon, the pancreas, the right gastroepiploic vein, the right gastroepiploic artery, the subpyloric artery, and the duodenum appear in the surgery image. In other words, the "information related to completion of resection" can include region information of at least one of the transverse mesocolon, the pancreas, the right gastroepiploic vein, the right gastroepiploic artery, the subpyloric artery, or the duodenum. Thus, in the gastrectomy, the state of resection of the resection target (for example, whether the resection of the resection target is completed or not, a ratio of the resected part to the resection target, or a ratio of the unresected part to the resection target) can be determined by the exposure degree of at least one of the transverse mesocolon, the pancreas, the right gastroepiploic vein, the right gastroepiploic artery, the subpyloric artery, or the duodenum.

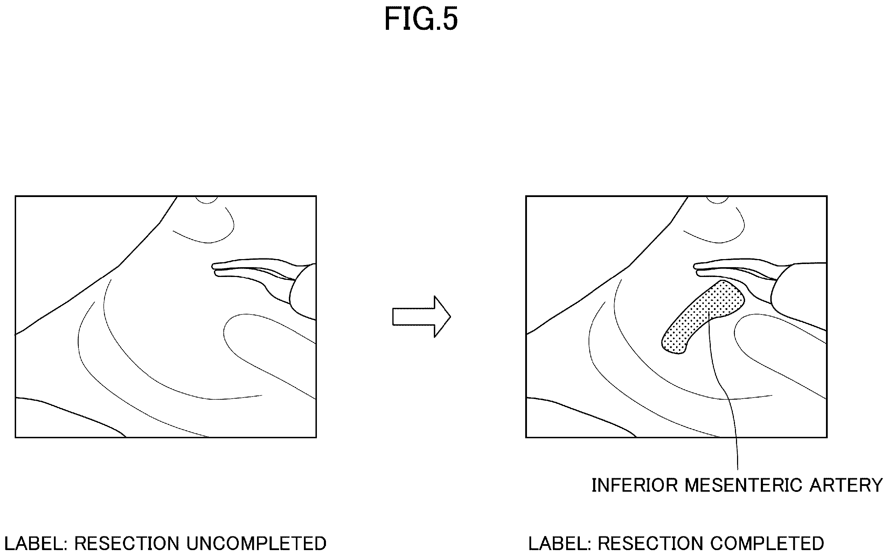

FIG. 5 is an example of the organs that appear in the surgery image when the resection target is resected according to an embodiment of the present invention. In a colectomy, when the resection target (tumor and lymph node) is resected, the abdominal aorta and the inferior mesenteric artery appear in the surgery image. In other words, the "information related to completion of resection" may include region information of at least one of the abdominal aorta or the inferior mesenteric artery. Thus, in the colectomy, the state of resection of the resection target (for example, whether the resection of the resection target is completed or not, a ratio of the resected part to the resection target, or a ratio of the unresected part to the resection target) can be determined by the exposure degree of at least one of the abdominal aorta or the inferior mesenteric artery.

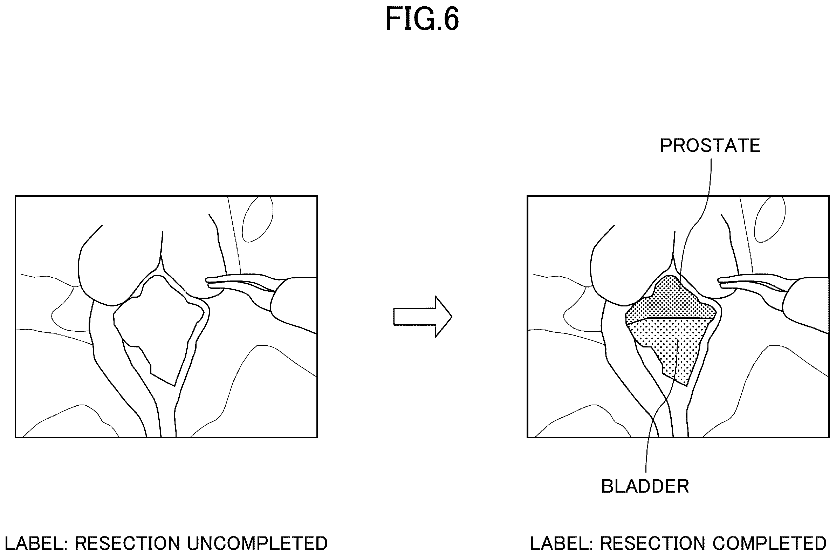

FIG. 6 is an example of the organs that appear in the surgery image when the resection target is resected according to an embodiment of the present invention. When the resection target is resected in the bladder neck transection of a prostatectomy, the prostate, the bladder, the urethra, the seminal vesicles, and the vas deferens appear in the surgery image. In other words, the "information related to completion of resection" can include region information of at least one of the prostate, the bladder, the urethra, the seminal vesicles, or the vas deferens. As described above, in the prostatectomy, the state of resection of the resection target (for example, whether the resection of the resection target is completed or not, or a ratio of the resected part to the resection target or a ratio of the unresected part to the resection target) can be determined by the exposure degree of at least one of the prostate, the bladder, the urethra, the seminal vesicle, or the vas deferens.

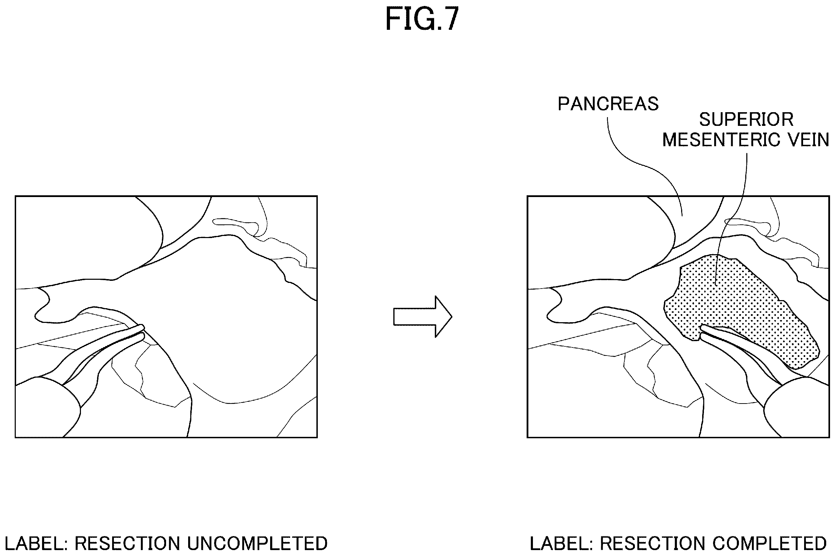

FIG. 7 is an example of the organs that appear in the surgery image when the resection target is resected according to an embodiment of the present invention. In a pancreatectomy, when the resection target (tumor and lymph node) is resected, the superior mesenteric vein appears in the surgery image. In other words, the "information related to completion of resection" can include the region information of the superior mesenteric vein. In this way, in the pancreatectomy, the state of resection of the resection target (for example, whether the resection of the resection target is completed or not completed, a ratio of the resected part to the resection target, or a ratio of the unresected part to the resection target) can be determined by the exposure degree of the superior mesenteric vein.

The description returns to FIG. 2. The resection state detector 103 detects a state of resection of the resection target using the model being trained.

### (Example 1)

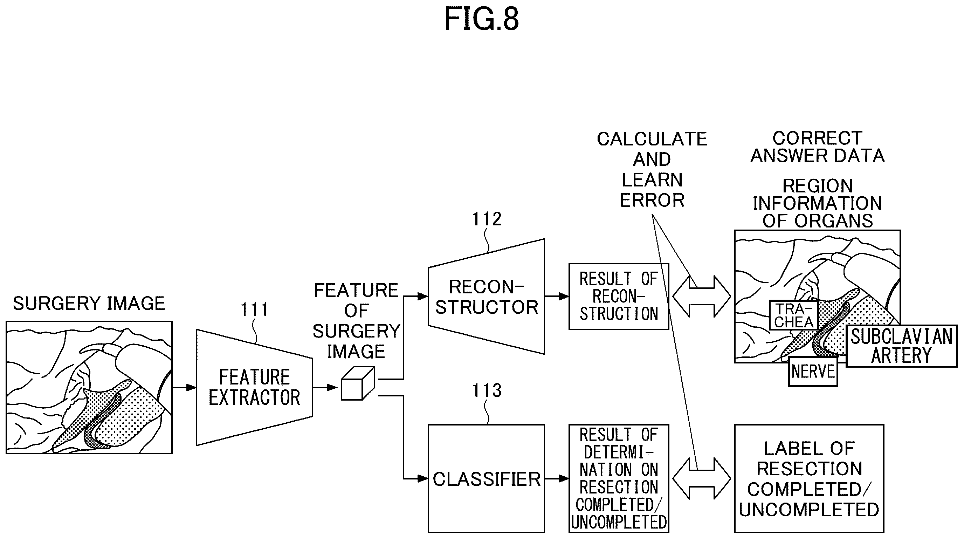

In Example 1 described with reference to FIG. 8, the resection state detector 103 inputs a surgery image acquired by the training data acquirer 102 to the model being trained (the feature extractor 111 and the reconstructor 112 in FIG. 8), and outputs region information of an organ. The resection state detector 103 inputs a surgery image acquired by the training data acquirer 102 to the model (the feature extractor 111 and the classifier 113 in FIG. 8) being trained, and outputs whether the resection of the resection target is completed or not.

### (Example 2)

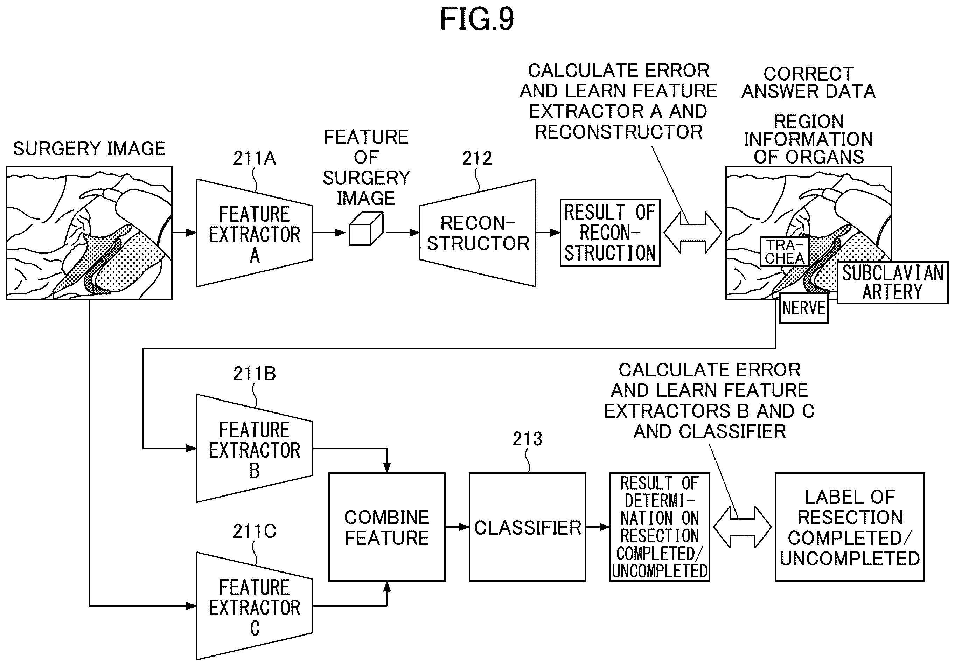

In Example 2 described with reference to FIG. 9, the resection state detector 103 inputs a surgery image acquired by the training data acquirer 102 to the model being trained (the feature extractor A 211A and the reconstructor 212 in FIG. 9), and outputs region information of an organ. The resection state detector 103 inputs the surgery image acquired by the training data acquirer 102 and the region information of the organ reconstructed by the reconstructor 212 to the model being trained (the feature extractor B 211B, the feature extractor C 211C, and the classifier 213 in FIG. 9), and outputs whether the resection of the resection target is completed or not.

The error calculator 104 calculates an error between a state of resection of the resection target detected from the surgery image by the resection state detector 103 and the information related to completion of resection for the surgery image acquired by the training data acquirer 102.

The parameter updater 105 updates parameters of the model being trained so that the error calculated by the error calculator 104 becomes small, and stores the updated parameters in the parameter storage 106.

The parameter storage 106 stores the parameters of the model updated by the parameter updater 105.

The machine learning process will be described in detail below with reference to FIG. 8 (Example 1) and FIG. 9 (Example 2).

FIG. 8 is a diagram for explaining machine learning according to an embodiment of the present invention (Example 1). The feature extractor 111 extracts a feature from a surgery image as training data, the reconstructor 112 reconstructs region information of an organ or a surgery image including region information of an organ from the feature, and the classifier 113 determines whether resection of a resection target is completed or not from the feature. In other words, in the inference in Example 1, the classifier 113 classifies the surgery image using the feature extracted by the feature extractor 111 at the time of machine learning. Each of these will be described below.

The feature extractor (also referred to as an "encoder") 111 extracts a feature of a surgery image from the surgery image. Specifically, the feature extractor 111 extracts a feature by reducing the dimension of a surgery image.

The reconstructor (also referred to as a "decoder") 112 reconstructs region information of an organ or a surgery image including region information of an organ based on the feature of the surgery image extracted by the feature extractor 111 (note that the reconstructed region information of an organ may be only the region information of an organ that appears at the time of resection of the resection target, or may include not only the region information of an organ that appears at the time of resection of the resection target but also the region information of other organs). An error between the result reconstructed by the reconstructor 112 and the region information of the organ of the training data (correct answer data) (i.e., the region information of the organ that appears in the surgery image when the resection target is resected) is calculated, and machine learning is performed (namely, the parameters are updated) so that the error becomes small.

The classifier 113 determines whether the surgery image is an image in which the resection of the resection target is completed or an image in which the resection of the resection target is not completed, based on the feature of the surgery image extracted by the feature extractor 111. An error between the result of the determination by the classifier 113 and the label of the training data (correct answer data) (i.e., the label indicating that the resection is completed or the label indicating that the resection is not completed) is calculated, and learning is performed so that the error is reduced (in other words, the parameters are updated).

FIG. 9 is a diagram for explaining the machine learning according to an embodiment of the present invention (Example 2). The feature extractor A 211A extracts a feature from the surgery image, and the reconstructor 212 reconstructs region information of an organ or a surgery image including region information of an organ from the feature. The feature extractor B 211B extracts a feature from the region information of an organ reconstructed by the reconstructor 212 or the surgery image including the region information of an organ. The feature extractor C 211C extracts a feature from the surgery image. The classifier 213 determines whether or not the resection of the resection target is completed from the feature obtained by combining the feature extracted by the feature extractor B 211B and the feature extracted by the feature extractor C 211C. In other words, in Example 2, at the time of inference, the feature extractor A 211A and the reconstructor 212 reconstruct region information of an organ from a surgery image, and the classifier 213 classifies the surgery image using a feature of the surgery image and a feature of the reconstructed region information of an organ. Each of these will be described below.

The feature extractor (also referred to as an "encoder") A 211A extracts a feature of a surgery image from the surgery image. Specifically, the feature extractor A 211A extracts a feature by reducing the dimension of a surgery image.

The reconstructor (also referred to as a "decoder") 212 reconstructs region information of an organ or a surgery image including region information of an organ based on the feature of the surgery image extracted by the feature extractor A 211A (note that the reconstructed region information of an organ may be only the region information of an organ that appears at the time of resection of the resection target, or may include not only the region information of an organ that appears at the time of resection of the resection target but also the region information of other organs). An error between the result reconstructed by the reconstructor 212 and the region information of the organ of the training data (correct answer data) (i.e., the region information of the organ that appears in the surgery image when the resection target is resected) is calculated, and machine learning is performed (namely, the parameters are updated) so that the error becomes small.

The feature extractor (also referred to as an "encoder") B 211B extracts a feature of region information of an organ from a region information of an organ reconstructed by the reconstructor 212. Specifically, the feature extractor B 211B extracts a feature by reducing the dimension of region information of an organ reconstructed by the reconstructor 212.

The feature extractor (also referred to as an "encoder") C 211C extracts a feature of a surgery image from the surgery image. Specifically, the feature extractor C 211C extracts a feature by reducing the dimension of a surgery image.

The classifier 213 determines whether the surgery image is an image in which the resection of the resection target is completed or an image in which the resection of the resection target is not completed, based on the feature obtained by combining the feature extracted by the feature extractor B 211B and the feature extracted by the feature extractor C 211C. An error between the result of the determination by the classifier 213 and the label of the training data (correct answer data) (i.e., the label indicating that the resection is completed or the label indicating that the resection is not completed) is calculated, and machine learning is performed so that the error is reduced (in other words, the parameters are updated).

By performing machine learning in this way, the surgery assistance apparatus 10 can reflect the degree of recognition of, and the positional relationship between, the resection target and the surrounding organs according to the progress of resection in the detection of a state of resection. For example, in the resection of a lymph node around the right recurrent laryngeal nerve, the state of resection of the lymph node can be determined based on the results of the segmentation of the right wall and the posterior wall of the trachea, the subclavian artery, and the recurrent laryngeal nerve. In other words, in the present invention, the state of resection of the resection target is detected using the result of segmentation of the anatomical structure and the frame information (i.e., the region information of each organ) as attention, and thus the information of each organ can contribute to the determination of which part should be resected and which part should be left without being resected.

The accuracy of the model inference was 66.6% in the case of machine learning without using the region information of an organ, and the accuracy of the model inference was 81.3% in the case of machine learning using the region information of an organ. In this way, by using the region information of an organ, the accuracy of the model inference can be improved.

The description returns to FIG. 2. The surgery image acquirer 107 acquires a surgery image (for example, a moving image) captured by the imaging apparatus 20 during surgery.

The resection state detector 108 detects the state of resection of the resection target based on the surgery image acquired by the surgery image acquirer 107. Specifically, the resection state detector 108 inputs the surgery image acquired by the surgery image acquirer 107 to a model (specifically, a model using the parameters stored in the parameter storage 106), and outputs the state of resection of the resection target (for example, whether resection of the resection target is completed or not, or a ratio of the resected part to the resection target or a ratio of the unresected part to the resection target). The probability that the resection of the resection target is completed (i.e., a ratio of the resected part) or the probability that the resection of the resection target is uncompleted (i.e., a ratio of the unresected part) may be output using a classification model, or the ratio of the resected part or the ratio of the unresected part may be output using a regression model.

The resection state notifier 109 provides notification about (for example, causes the display apparatus 30 to display) the state of resection of the resection target detected by the resection state detector 108 in real time. For example, the resection state notifier 109 provides notification that the resection of the resection target is completed (in other words, indicates whether the resection is completed or not by two values), or provides notification of the ratio of the resected part to the resection target or the ratio of the unresected part to the resection target (in other words, indicates the state of resection by percentage). For example, the resection state notifier 109 displays the state of resection of the resection target on the surgery image acquired by the surgery image acquirer 107. For example, the resection state notifier 109 displays "resection completed" or the like.

### <Method of the Process>

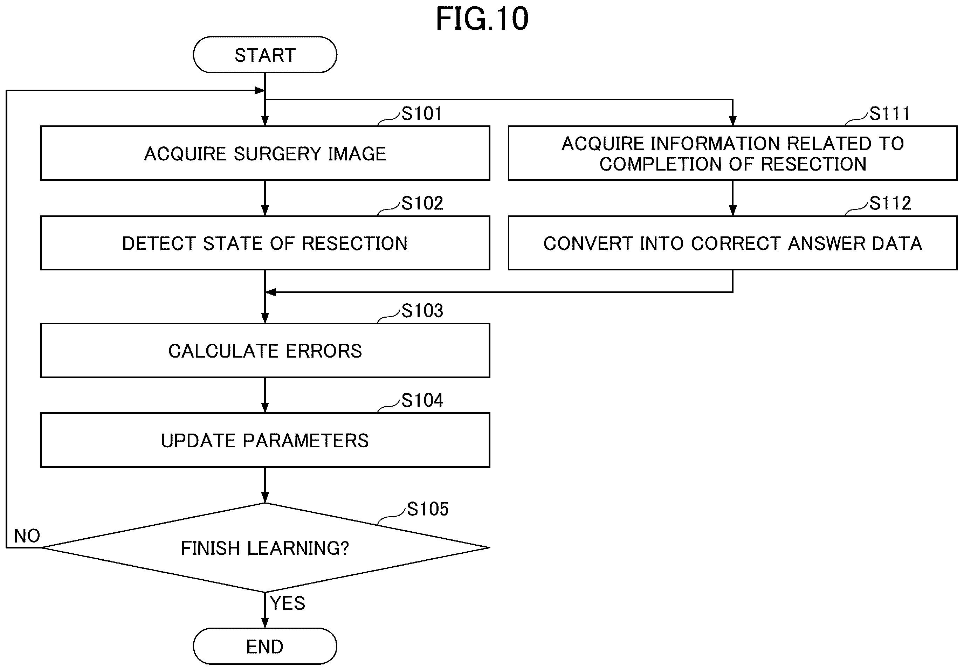

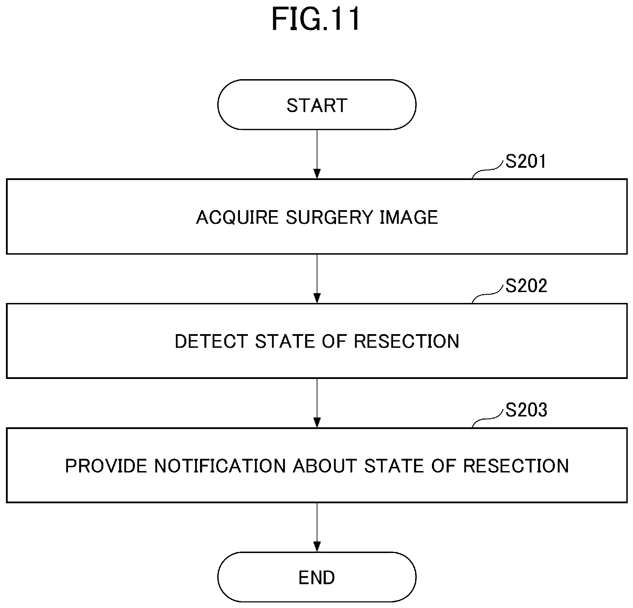

Hereinafter, a method of the learning process will be described with reference to FIG. 10, and a method of the resection state notification process will be described with reference to FIG. 11.

FIG. 10 is a flowchart of a machine learning process according to an embodiment of the present invention.

In step 101 (S101), the surgery assistance apparatus 10 acquires a surgery image which is training data.

In step 102 (S102), the surgery assistance apparatus 10 detects a state of resection of a resection target using a machine learning model being trained.

In Example 1 described with reference to FIG. 8, the surgery assistance apparatus 10 inputs the surgery image acquired in S101 to the model (the feature extractor 111 and the reconstructor 112 in FIG. 8) being trained, and outputs region information of an organ. The surgery assistance apparatus 10 inputs the surgery image acquired in S101 to the model (the feature extractor 111 and the classifier 113 of FIG. 8) being trained, and outputs whether the resection of the resection target is completed or not.

In Example 2 described with reference to FIG. 9, the surgery assistance apparatus 10 inputs the surgery image acquired in S101 to the model (the feature extractor A 211A and the reconstructor 212 in FIG. 9) being trained, and outputs region information of an organ. The resection state detector 10 inputs the surgery image acquired in S101 and the region information of an organ reconstructed by the reconstructor 212 to the model being trained (the feature extractor B 211B, the feature extractor C 211C, and the classifier 213 in FIG. 9), and outputs whether the resection of the resection target is completed or not.

In step 111 (S111), the surgery assistance apparatus 10 acquires information related to completion of resection (in detail, region information of an organ that appears in the surgery image when the resection target is resected, and a label indicating whether the surgery image is an image in which the resection of the resection target is completed or an image in which the resection of the resection target is not completed) for the surgery image that is training data (i.e., the surgery image acquired in S101).

In step 112 (S112), the surgery assistance apparatus 10 converts the data acquired in S111 into correct answer data. Specifically, the surgery assistance apparatus 10 arranges the data acquired in S111 in the format of an output of the model.

In step 103 (S103), the surgery assistance apparatus 10 calculates errors between the state of resection of the resection target detected in S102 and the correct answer data in S112.

In step 104 (S104), the surgery assistance apparatus 10 updates the parameters of the model being trained so that the errors calculated in S103 become small, and stores the updated parameters in the parameter storage 106.

In step 105 (S105), the surgery assistance apparatus 10 determines whether or not to end the training. If the training is not to be terminated, the process returns to S101 and S111 to acquire a next set of training data.

FIG. 11 is a flowchart of the resection state notification process according to an embodiment of the present invention.

In step 201 (S201), the surgery assistance apparatus 10 acquires a surgery image (for example, a moving image) captured by the imaging apparatus 20 during surgery.

In step 202 (S202), the surgery assistance apparatus 10 detects the state of resection of the resection target based on the surgery image acquired in S201. Specifically, the surgery assistance apparatus 10 inputs the surgery image acquired in S201 to the model generated in FIG. 10, and outputs the state of resection of the resection target (for example, whether resection of the resection target is completed or not, a ratio of the resected part to the resection target, or a ratio of the unresected part to the resection target).

In the first embodiment described with reference to FIG. 8, the classifier 113 classifies the surgery image using the feature extracted by the feature extractor 111 at the time of machine learning.

In other words, in Example 2 described with reference to FIG. 9, at the time of inference, the feature extractor A 211A and the reconstructor 212 reconstruct region information of an organ from a surgery image, and the classifier 213 classifies the surgery image using a feature of the surgery image and a feature of the reconstructed region information of an organ.

In step 203 (S203), the surgery assistance apparatus 10 provides notification about (for example, causes the display 30 to display) the state of resection of the resection target detected in S202 in real time. For example, the surgery assistance apparatus 10 provides notification that the resection of the resection target is completed, or provides notification of a ratio of the resected part to the resection target or a ratio of the unresected part to the resection target. For example, the surgery assistance apparatus 10 displays the state of resection of the resection target on the surgery image acquired in S201.

### <Effects>

In one embodiment of the present invention, during a surgical operation, when resection of cancerous tissue such as a primary lesion or a lymph node is completed, notification of completion of the procedure is provided in real time. A clear indication of the notification of the completion of the procedure in the surgery image enables the doctor to sufficiently resect the lymph node while an increase in complications is avoided.

Other embodiments will be described below. A plurality of embodiments described below may be implemented in combination.

### (Notification of Unresected Site)

When resection of the resection target is not completed, the surgery assistance apparatus 10 can provide notification of the unresected part of the resection target. Specifically, the surgery assistance apparatus 10 specifies a part that has not been resected in the resection target in the surgery image captured by the imaging apparatus 20 during surgery, and provides notification of the specified portion (for example, the surgery assistance apparatus 10 can specify a part which has not been resected in the resection target on a rule basis, on the basis of the segmentation information output from the reconstructor 112. For example, in the case where "nerves (an example of organs that appear in the surgery image when the resection target is resected)" do not appear in the surgery image (in other words, in the case where the segmentation information of "nerves" is not acquired), a part where "nerves" should appear can be specified as an unresected part. For example, the surgery assistance apparatus 10 notifies the operator when the operator proceeds from a resection step to the next step in surgery or when the operator is in a resection step. For example, the surgery assistance apparatus 10 causes the display apparatus 30 to display "The resection around the trachea is insufficient" or the like.

Thus, providing notification about which part is not completely resected can let the operator know the procedure to be performed next.

### (Notification of Uncompleted Resection)

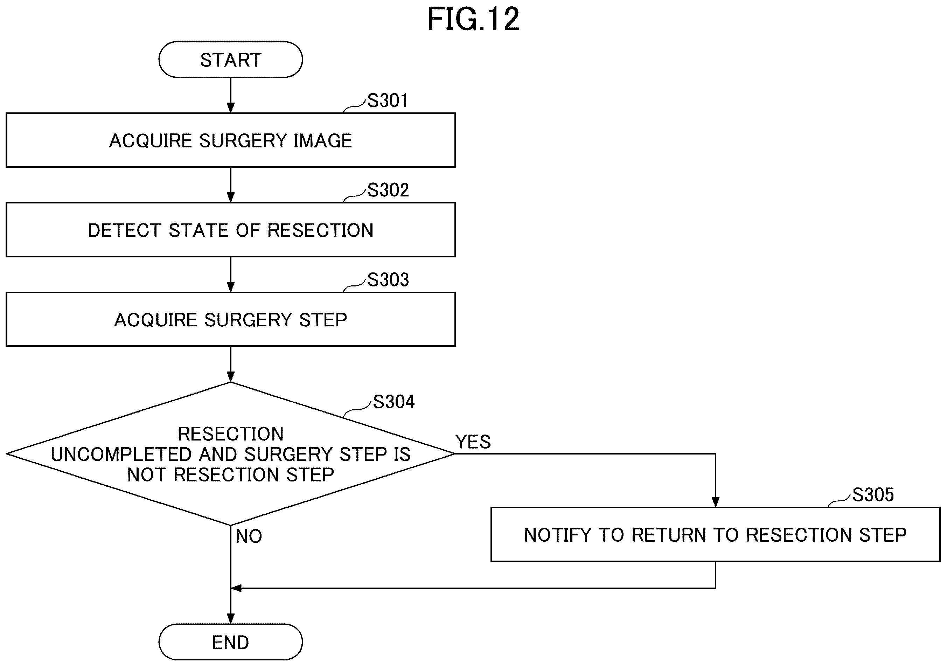

When resection of the resection target is not completed, if the operator proceeds from the resection step to the next step of the surgery, the surgery assistance apparatus 10 can notify the operator to return to the resection step. The uncompleted resection notification process will be described below with reference to FIG. 12. Hereinafter, assume that each step (for example, a resection step) during surgery is managed.

FIG. 12 is a flowchart of an uncompleted resection notification process according to an embodiment of the present invention.

In step 301 (S301), the surgery assistance apparatus 10 acquires a surgery image captured by imaging apparatus 20 during surgery.

In step 302 (S302), the surgery assistance apparatus 10 detects a state of resection of the resection target based on the surgery image acquired in S301.

In step 303 (S303), the surgery assistance apparatus 10 acquires information indicating which of the steps in the surgery the current step is.

In step 304 (S304), the surgery assistance apparatus 10 determines whether or not the resection of the resection target is not completed and the current step is not the resection step. If the resection of the resection target is not completed and the current step is not a resection step, the process proceeds to step 305; otherwise, the process ends.

In step 305 (S305), the surgery assistance apparatus 10 notifies the operator to return to the resection step. For example, the surgery assistance apparatus 10 causes the display apparatus 30 to display "Return to the previous procedure of resection" or the like.

In this way, notifying the operator to return to the resection step can prevent the procedure from ending before sufficient resection is completed.

### (Management of Resection Time)

When a certain time or more has elapsed after a resection step of the surgery is started, if resection of the resection target is not completed, the surgery assistance apparatus 10 can provide notification that the certain time or more has elapsed in the resection step of the surgery. For example, the predetermined time may be an average time of past data, or may be a time exceeding the average time of past data +2SD (standard deviations). For example, the surgery assistance apparatus 10 causes the display apparatus 30 to display "A certain period of time has elapsed since the start of resection" or the like.

Managing the time of the resection step can predict complications and can provide notification of the timing of changing the operator to a more skillful operator, in addition to the prevention of an end of the procedure before sufficient resection is completed.

### (Evaluation of Procedure)

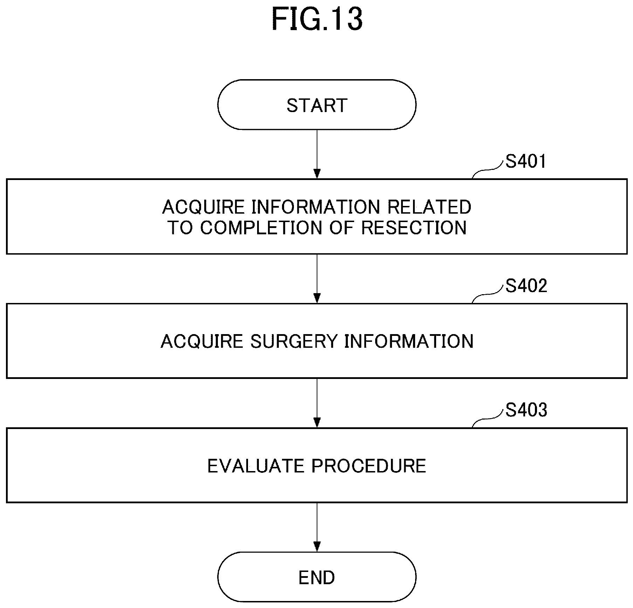

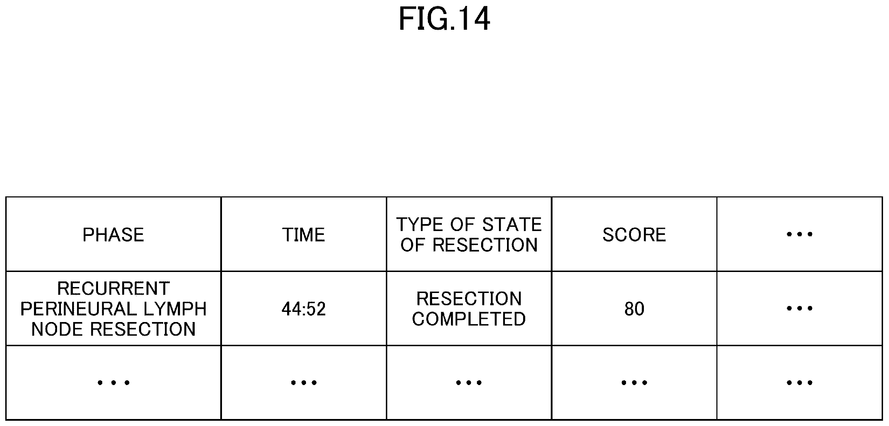

The surgery assistance apparatus 10 can evaluate the procedure of the operator, such as a surgeon or an endoscopist, based on the information related to completion of resection that is output by inputting a surgery image to the model. Hereinafter, the evaluation of the procedure will be described with reference to FIGS. 13 and 14.

FIG. 13 is a flowchart of an evaluation process according to an embodiment of the present invention.

In step 401 (S401), the surgery assistance apparatus 10 acquires information related to completion of resection that is output by inputting a surgery image to the model.

In step 402 (S402), the surgery assistance apparatus 10 acquires information related to surgery. The information related to surgery may be an entire surgery time, a surgery time in each phase, data obtained by recognizing the movement of the instrument or the operator, or the like.

In step 403 (S403), the surgery assistance apparatus 10 evaluates the procedure performed by the operator based on the information acquired in S401 and S402. For example, the surgery assistance apparatus 10 may evaluate the procedure of the operator, using machine learning.

FIG. 14 is an example of information on which evaluation is based and of evaluation results, according to an embodiment of the present invention. For example, the information on which the evaluation is based is the time of each phase of the surgery (for example, recurrent perineural lymph node resection), the type of state of resection (e.g., completion of resection), and the like. For example, the evaluation result may be a score.

Through evaluating the procedure, it is possible to perform feedback of the procedure of the surgery after the surgery and to use the procedure for surgery education.

### <Hardware Configuration>

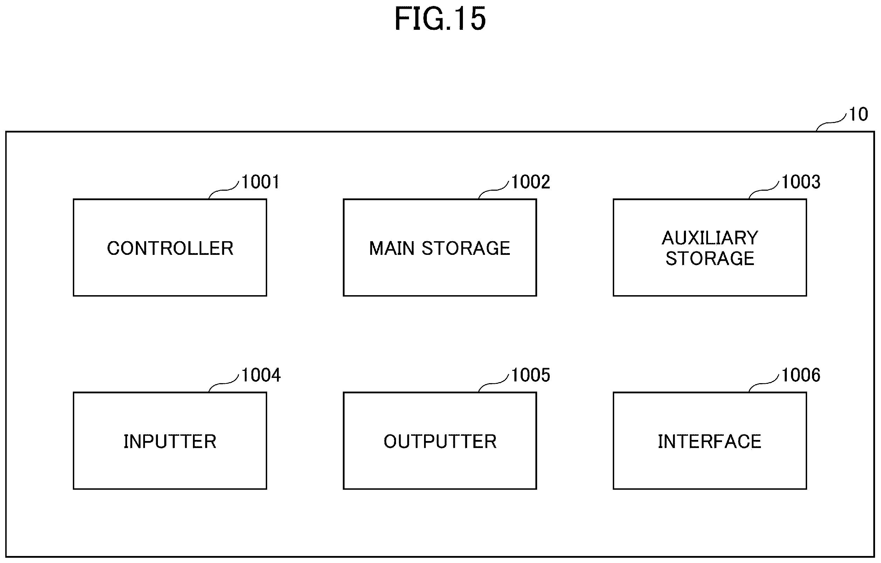

FIG. 15 is a hardware configuration diagram of the surgery assistance apparatus 10 according to an embodiment of the present invention. The surgery assistance apparatus 10 can include a controller 1001, a main storage 1002, an auxiliary storage 1003, an inputter 1004, an outputter 1005, and an interface 1006. Each of these will be described below.

The controller 1001 is a processor (for example, a central processing unit (CPU), a graphics processing unit (GPU), or the like) that executes various programs installed in the auxiliary storage 1003.

The main storage 1002 includes a nonvolatile memory (read only memory (ROM)) and a volatile memory (random access memory (RAM)). The ROM stores various programs, data, and the like necessary for the controller 1001 to execute various programs installed in the auxiliary storage 1003. The RAM provides a work area in which various programs installed in the auxiliary storage 1003 are loaded when the controller 1001 executes the programs.

The auxiliary storage 1003 is an auxiliary storage device that stores various programs and information used when the various programs are executed.

The inputter 1004 is an input device through which a user of the surgery assistance apparatus 10 inputs various instructions to the surgery assistance apparatus 10.

The outputter 1005 is an output device that outputs an internal state of the surgery assistance apparatus 10 and the like.

The interface 1006 is a communication device for connecting to a network and communicating with other devices.

Although the embodiments of the present invention have been described in detail, the present invention is not limited to the above-described specific embodiments, and various modifications and changes can be made within the scope of the gist of the present invention described in the claims.

This international application claims priority based on Japanese Patent Application No. 2022-143971 filed on September 9, 2022, and the entire contents of Japanese Patent Application No. 2022-143971 are incorporated herein by reference.

### REFERENCE SIGN LIST

- 1: Surgery assistance system
- 10: Surgery assistance apparatus
- 20: Imaging apparatus
- 30: Display apparatus
- 11: Doctor
- 12: Patient
- 101: Training data storage
- 102: Training data acquirer
- 103: Resection state detector
- 104: Error calculator
- 105: Parameter updater
- 106: Parameter storage
- 107: Surgery image acquirer
- 108: Resection state detector
- 109: Resection state notifier
- 111: Feature extractor
- 112: Reconstructor
- 113: Classifier
- 211A: Feature extractor A
- 211B: Feature extractor B
- 211C: Feature extractor C
- 212: Reconstructor
- 213: Classifier
- 1001: Controller
- 1002: Main storage
- 1003: Auxiliary storage
- 1004: Inputter
- 1005: Outputter
- 1006: Interface

## Claims

1. A program that causes a computer to:
acquire a surgery image in which a surgical site is imaged during surgery for resecting a resection target;
detect a state of resection of the resection target from the acquired surgery image by using a machine learning model generated by training with training data in which a surgery image is an input and information related to completion of resection is an output; and
provide notification about a state of resection of the resection target, wherein
the information related to completion of resection includes region information of an organ that appears in the surgery image when the resection target is resected.

2. The program according to claim 1, wherein
the providing of notification about the state of resection of the resection target is providing notification that resection of the resection target is completed, or providing notification of a ratio of a resected part to the resection target or a ratio of an unresected part to the resection target.

3. The program according to claim 1 or **2,** wherein the program further causes the computer to cause the state of resection of the resection target to be displayed on the acquired surgery image.

4. The program according to claim 1 or 2, wherein
the program further causes the computer to, if resection of the resection target is not completed, provide notification of an unresected site in the resection target.

5. The program according to claim 1 or 2, wherein
the program further causes the computer to, when an operator proceeds from a resection step to a next step of the surgery while resection of the resection target is not completed, notify the operator to return to the resection step.

6. The program according to claim 1 or 2, wherein
the program further causes the computer to, when a predetermined time or more has elapsed after the resection step of the surgery is started while resection of the resection target is not completed, provide notification that the resection step of the surgery has been performed for a predetermined time or more.

7. The program according to claim 1 or 2, wherein
the program further causes the computer to evaluate a procedure of the surgery performed by the operator based on information related to completion of resection that is output in response to an input of the acquired surgery image to the machine learning model.

8. A surgery assistance apparatus, comprising:
a surgery image acquirer configured to acquire a surgery image in which a surgical site is imaged during surgery for resecting a resection target;
a resection state detector configured to detect a state of resection of the resection target from the acquired surgery image by using a machine learning model generated by training with training data in which a surgery image is an input and information related to completion of resection is an output; and
a resection state notifier configured to provide notification about a state of resection of the resection target, wherein
the information related to completion of resection includes region information of an organ that appears in the surgery image when the resection target is resected.

9. A method performed by a surgery assistance apparatus, the method comprising:
a step of acquiring a surgery image in which a surgical site is imaged during surgery for resecting a resection target;
a step of detecting a state of resection of the resection target from the acquired surgery image by using a machine learning model generated by training with training data in which a surgery image is an input and information related to completion of resection is an output; and
a step of providing notification about a state of resection of the resection target, wherein
the information related to completion of resection includes region information of an organ that appears in the surgery image when the resection target is resected.
